# EUROPEAN PATENT APPLICATION

(11) **EP 1 887 361 A1**
(43) Date of publication of application: **13.02.2008**
(21) Application number: 06291277.9
(22) Date of filing: 07.08.2006
(51) Int. Cl.: G01N 33/68

(54) **Method for the prediction of vascular events**

(71) Applicant: Bio-Rad Pasteur, 92430 Marnes-la-Coquette (FR)
(72) Inventor: Villard-Saussine, Sylvie, 75014 Paris (FR); Le Moal, Estelle, 92800 Puteaux (FR); Larue, Catherine, 92420 Vaucresson (FR); Giuliani, Isabelle, 92380 Garches (FR)
(74) Representative: Colombet, Alain André

(57) **Abstract**

The present invention relates to a method for the prognosis of a vascular event in a patient suspected of being at risk for a vascular event, said patient presenting:
- no elevation of the ST segment as seen on an electrocardiogram, and/or
- a normal level of at least one myocardial necrosis marker,
wherein the presence and/or levels of at least two different biochemical markers are measured in a biological sample of said patient, whereby the probability that the patient will experience a vascular event is deduced from the measured presence and/or levels of the biochemical markers such as MPO, sCD40L, IL-6, PaPP-A, CRP, D-dimer, troponin, and proBNP.

## Description

The present invention relates to a method for predicting the occurrence of vascular events in patients suspected of being at risk for such events.

Each year, millions of patients present to hospitals with symptoms, such as chest pain, indicative of acute coronary syndrome (ACS). ACS refers to a variety of clinical symptoms caused by acute myocardial ischemia. Identification of patients having ACS is of tremendous importance, due to their higher risk for cardiac death or ischemic complications.

The current protocols for assessing the status of patients suspected of ACS usually include performing an electrocardiogram (ECG) and determining the blood level of a myocardial necrosis marker, commonly cardiac Troponin I or T (cTnl or cTnT) or creatine kinase (CK).

Among the patients tested, those presenting no elevation of the ST segment on the electrocardiogram recording and normal levels of the myocardial necrosis marker are particularly difficult to deal with. On one hand, admission of these patients is most often unnecessary because of their limited risk of enduring adverse vascular events, with unnecessary admissions bearing high costs on health protection systems. On the other hand, not all of these patients are risk-free, notably because the presentation of an ACS is often atypical, and it is estimated that as much as 1-2% of patients who are sent home have an adverse vascular event afterwards, such as myocardial infarction (Apple et al. (2005) Clinical Chemistry 51:5).

As such, among patients suspected of being at risk for a vascular event and who present with no elevation of the ST segment and normal levels of a myocardial necrosis marker, there is an important need for a method to help discriminate between risk-free patients and those effectively at risk of undergoing an adverse vascular event.

Therefore, it is an object of the present invention to provide a method for the prognosis of vascular events in patients who present no elevation of the ST segment and normal levels of a myocardial necrosis marker.

It is another object of the invention to provide a reliable method for diagnosing ACS among these patients.

It is a further object of the invention to provide kits for implementing the above methods.

The present invention arises from the demonstration, by the Inventors, that the probability of future vascular events in patients suspected of being at risk for such events can be determined by:
- measuring the presence and/or the level of at least two different biochemical markers selected from the group consisting of the following classes of markers:
   - atherosclerotic plaque instability markers,
   - atherosclerotic plaque disruption markers,
   - coronary inflammation markers,
   - pre-thrombotic status markers,
   - thrombosis markers,
   - ischemic markers,
   - necrosis markers, and
   - myocardial dysfunction markers,
- deducing from the measured presence and/or level of said biochemical markers, the probability that the patient will experience a vascular event.

### Summary of the Invention

The present invention relates to a method for the prognosis of a vascular event in a patient suspected of being at risk for a vascular event, said patient presenting in particular:
- no elevation of the ST segment as seen on an electrocardiogram, and/or
- a normal level of at least one marker of myocardial necrosis marker,
wherein the presence and/or the level of at least two different biochemical markers, in particular at least three, more particularly at least four, and even more particularly at least five different biochemical markers, is measured in a biological sample of said patient, said biochemical markers being selected from the group consisting of the following classes of markers:
- atherosclerotic plaque instability markers,
- atherosclerotic plaque disruption markers,
- coronary inflammation markers,
- pre-thrombotic status markers,
- thrombosis markers,
- ischemic markers,
- necrosis markers, and
- myocardial dysfunction markers,
whereby the probability that the patient will experience a vascular event is deduced from the measured presence and/or level of the biochemical markers.

The present invention also relates to a method for diagnosing acute coronary syndrome in a patient presenting in particular:
- no elevation of the ST segment as seen on an electrocardiogram, and/or
- a normal level of at least one myocardial necrosis marker,
comprising measuring the presence and/or the level of at least two different biochemical markers, in particular at least three, more particularly at least four, and even more particularly at least five different biochemical markers, in a biological sample of said patient, said biochemical markers being selected from the group consisting of the following classes of markers:
- atherosclerotic plaque instability markers,
- atherosclerotic plaque disruption markers,
- coronary inflammation markers,
- pre-thrombotic status markers,
- thrombosis markers,
- ischemic markers,
- myocardial necrosis markers, and
- myocardial dysfunction markers,
whereby it is determined if said patient suffers from ACS.

In an embodiment of the above-defined prognosis and diagnosis methods the patient presents:
- no elevation of the ST segment as seen on an electrocardiogram, and
- a normal level of at least one myocardial necrosis marker.

In another embodiment of the above-defined prognosis and diagnosis methods, the patient further presents a chest pain.

In another embodiment of the above defined prognosis and diagnosis methods the presence or the level of the myocardial necrosis marker the level of which is normal in said patient to which the prognosis or diagnosis methods are applied, is used for deducing the probability that the patient will experience a vascular event or for determining if said patient suffers from ACS.

In yet another embodiment of the above-defined prognosis and diagnosis methods, the patient presents a normal cardiac Troponin level, in particular a normal cardiac Troponin I (cTnl) level, and/or a normal CK level.

In a further embodiment of the above-defined prognosis and diagnosis methods, the patient presents no myocardial necrosis.

The present invention also relates to a kit intended for diagnosing ACS or for the prognosis of a vascular event in a patient, said kit comprising at least three, in particular three or four, specific ligands respectively to at least three, in particular three or four, biochemical markers selected from the group consisting of the following classes of markers:
- atherosclerotic plaque instability markers,
- atherosclerotic plaque disruption markers,
- coronary inflammation markers,
- pre-thrombotic status markers,
- thrombosis markers,
- ischemic markers,
- myocardial necrosis markers,
- myocardial dysfunction markers.

In an embodiment, the above defined kit comprises one specific ligand to cardiac Troponin, in particular to cardiac Troponin I.

### Detailed description of the invention

As intended herein "prognosis" relates to methods for predicting the outcome of a pathology or for predicting the probability or likeliness that an adverse event will occur in a patient.

As intended herein "vascular event" relates to a vascular-related, in particular a cardiovascular-related, health-threatening or pathological event in a patient organism. Vascular events according to the invention are in particular selected from the group consisting of vascular-related death, myocardial infarction, congestive-heart failure and vascular-related hospitalization, revascularization procedure.

As intended herein "diagnosis" relates to method for determining the pathology afflicting a patient.

As intended herein acute coronary syndrome (ACS) relates to a variety of clinical symptoms caused by acute myocardial ischemia. It is notably defined in Apple et al. (2005) Clinical Chem 51:810-824.

As intended herein "no elevation of the ST segment as seen on an electrocardiogram" means that the patient's electrocardiogram either presents a depression of the ST segment or a normal ST segment.

As intended herein a "normal level" of a marker means that the concentration of the marker in the biological sample is within the norm cut-off values for that marker. The norm is dependant on the biological sample type and on the method used for measuring the concentration of the marker in the biological sample. Norms for a given marker are well known to the man skilled in the art. In particular, a normal level for a given marker means that the marker is substantially absent, that is the level measured for that marker is below a cut-off value defining the minimal level for that marker to be considered present.

As intended herein a "myocardial necrosis marker" relates to a macromolecule which is released from myocardial tissues upon myocardial necrosis, notably after myocardial ischemia. Myocardial necrosis marker notably encompasses cardiac Troponin, in particular cardiac Troponin I and T (cTnl and cTnT), myoglobin, as well as creatine kinase (CK). As regards CK it is preferred that the MB isoform is measured.

Preferably, to be regarded as normal, measured levels for cardiac Troponin I should not exceed the 99^{th} percentile of values for a reference group during at least 24 hours.

Preferably also, to be regarded as normal, measured levels for the MB isoform of CK should not exceed the 99^{th} percentile of values for a gender-specific reference control group on two successive samples.

As intended herein the levels of the markers are measured using any method liable to yield the concentration of the marker in the biological sample. Such a method notably encompass mass spectroscopy (in particular for measuring CK-MB levels), but also ligand-based methods such as immunological method.

In particular the levels of the biochemical markers are measured using ligands specific for said biochemical markers.

A "ligand" relates to a molecule which has binding affinity towards a marker. In particular it is preferred that the ligand binds to the marker in a specific manner, that is the ligand preferentially binds to the marker it is specific for as compared to other components of the biological sample.

The specific ligands are in particular selected from the group consisting of:
- polyclonal, monoclonal and recombinant antibodies, or fragments thereof, such as Fab fragments, F(ab')₂ fragments and scFv fragments,
- phage antibodies (PhAbs), or
- aptamers.

As intended herein the expression "biological sample" encompasses all samples which can be taken from a patient, such as tissue biopsies or samples of body fluids. However, it is preferred in the Invention that the samples are blood-based samples, such as whole-blood samples, serum samples or plasma samples. In case of blood based samples an anticoagulant, such as EDTA, citrate or heparin, can be added.

Furthermore, the levels of the markers can be measured from different samples taken from a same patient or from a unique sample.

Moreover, the presence or the levels of markers in the samples can be determined individually, in simplex assays, or optionally, the levels of some or all of the markers can be determined simultaneously, in multiplex assays.

The presence or the level of a marker can be determined once or repeatedly, notably according to a predefined time-course.

It is preferred that the presence or the level of a marker is determined using an automated assay system.
As intended herein:
- atherosclerotic plaque instability markers relate to markers of which the presence or the level is altered in case of instable atherosclerotic plaque. In particular such markers are present or their level increases in case of instable atherosclerotic plaque;
- atherosclerotic plaque disruption markers relate to markers of which the presence or the level is altered in case of atherosclerotic plaque disruption. In particular such markers are present or their level increases in case of atherosclerotic plaque disruption;
- coronary inflammation markers relate to markers of which the presence or the level is altered in case of coronary inflammation. In particular such markers are present or their level increases in case of coronary inflammation;
- pre-thrombotic status markers relate to markers of which the presence or the level is altered in case of pre-thrombosis. In particular such markers are present or their level increases in case of pre-thrombosis;
- thrombosis markers relate to markers of which the presence or the level is altered in case of thrombosis. In particular such markers are present or their level increases in case of thrombosis;
- ischemic markers relate to markers of which the presence or the level is altered in case of myocardial ischemia. In particular such markers are present or their level increases in case of myocardial ischemia;
- myocardial necrosis markers relate to markers of which the presence or the level is altered in case of myocardial necrosis. In particular such markers are present or their level increases in case of myocardial necrosis;
- myocardial dysfunction markers relate to markers of which the presence or the level is altered in case of myocardial dysfunction. In particular such markers are present or their level increases in case of myocardial dysfunction.

Such markers are notably described in Apple et al. (2005) Clin. Chem. 51:810-824.

In a preferred embodiment of the above-defined prognosis and diagnosis methods, the biochemical markers do not belong to the same class of markers.

In another preferred embodiment of the above-defined prognosis and diagnosis methods, the following combinations of markers are used:

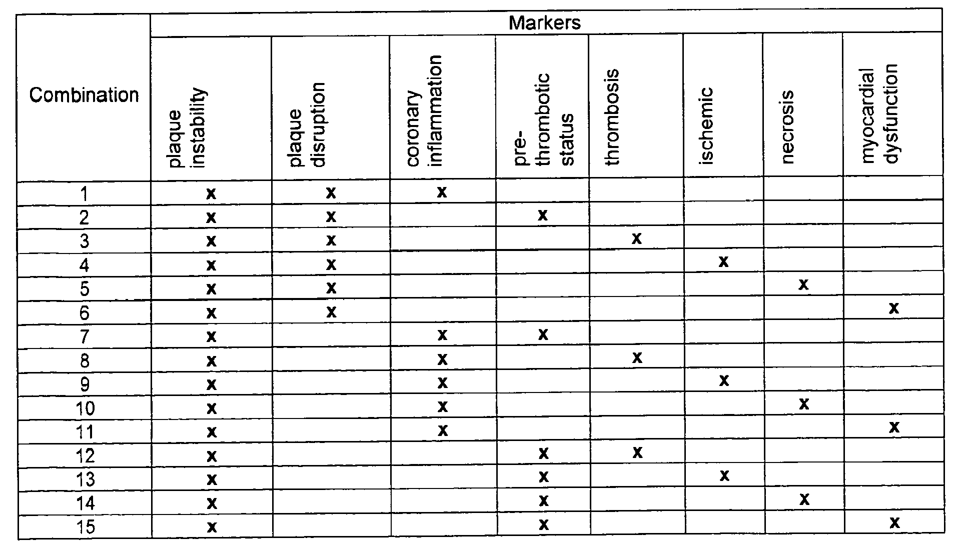

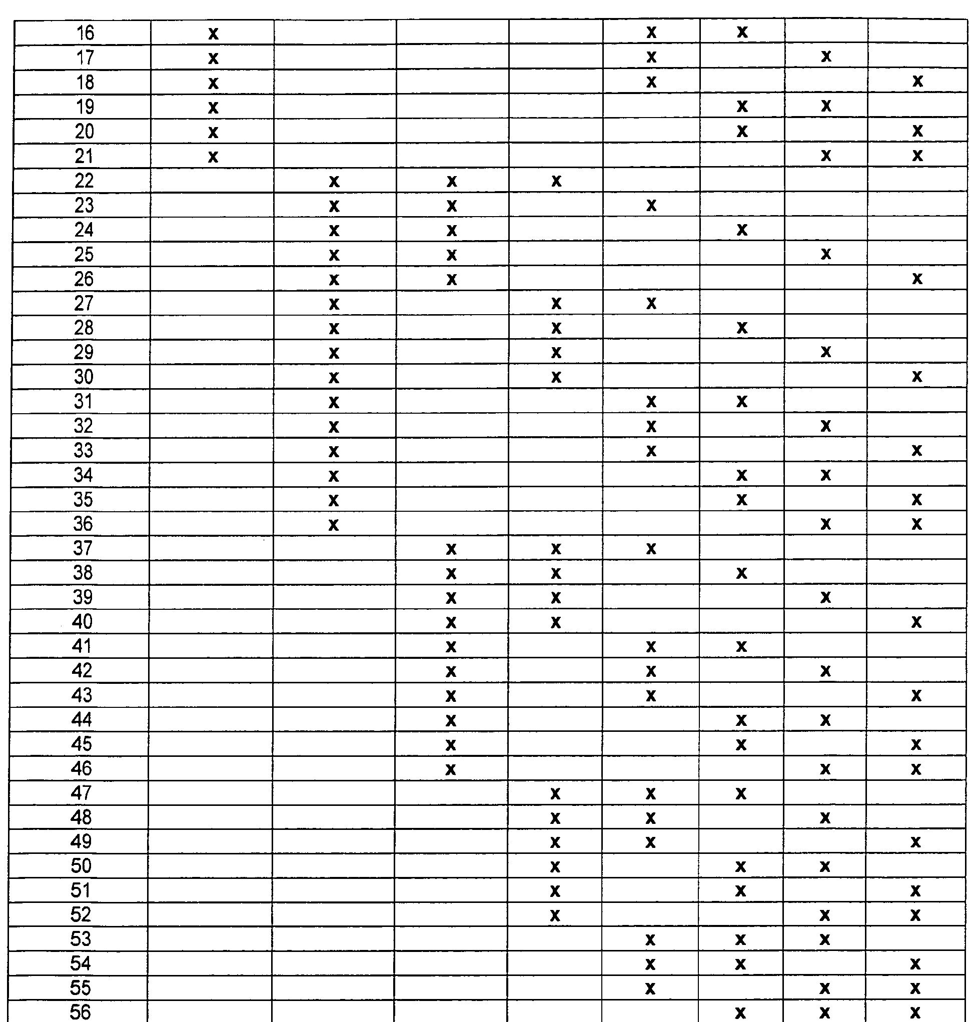

According to a particularly preferred embodiment of the above-defined prognosis and diagnosis methods:
- the atherosclerotic plaque instability marker is selected from the group consisting of MPO, sCD40L, IL-6, PaPP-A and Choline, in particular whole blood Choline;
- the atherosclerotic plaque disruption marker is selected from the group consisting of MPO, sCD40L, MMPs, Choline, in particular whole blood Choline, and PaPP-A;
- the coronary inflammation marker is selected from the group consisting of MPO, sCD40L, CRP and IL-6;
- the pre-thrombotic status marker is selected from the group consisting of sCD40L and fibrinogen;
- the thrombosis marker is selected from the group consisting of D-dimers;
- the ischemic marker is selected from the group consisting of IMA, cTnl, cTnT, and FABP;
- the myocardial necrosis marker is selected from the group consisting of cTnl, cTnT, CK, Myoglobin and FABP;
- the myocardial dysfunction marker is selected from the group consisting of

BNP, NT-proBNP and proBNP. MPO (myeloperoxydase) is notably described in Baldus et al., Circ. 2003; 108:1440-1445, Brennan et al., N Engl J Med 2003; 349:1595-604, Brennan et al., Current opinion in lipidology 2003; 14:353-359, Nambi et al., Curr Ather Reports 2005, 7:127-131, Nicholls et al., Arterioscler Thromb Vasc Biol. 2005; 25:1-9 and Zhang et al. JAMA 2001; 286:2136-2142.

MMPs (matrix metalloproteinases), in particular MMP-9, are notably described in Jones et al., Cardiovasc Res 2003; 59:812-23.

Choline is notably described in Apple et al., Clin. Chem. 2005; 51:810-824. sCD40L (soluble form of CD40 ligand) is notably described in Aukrust et al., Circ. 1999; 100:614-620; Heeschen et al., N Engl J Med 2003; 348:1104-11; Peng et al., Clin Chim Acta. 2002; 319:19-26; Tayebjee et al., Am J Cardiol 2005; 96:339-345; Varo et al., Circ. 2003; 108:1049-1052.

IL-6 (interleukin 6) is notably described in Aggarwal et al. Journal of Thrombosis and Thrombolysis 2003 ; 15: 25-31, 2003; Biasucci et al. Circ. 1999; 99:855-860; Ikeda et al. Clin. Cardiol. 2001;24:701-704; Lubrano et al., J of Clin Lab Anal 2005; 19:110-114; Ridker et al., N Engl J Med. 2000; 342:836-43.

PaPP-A (pregnancy associated plasma protein A) is notably described in Bayes-Genis et al., N Engl J Med 2001; 345:1022-9; Heeschen et al., J Am Coll Cardiol 2005; 45:229-37; Khosravi et al., Clinical Biochemistry 2002; 35:531-538; Laterza et al., Clin Chim Acta 2004; 348:163-169; Liu et al., Chin med j. 2005; 118:1827-1829. Lund et al., Circulation 2003; 108:1924-1926. Qin et al., Scand Cardiovasc J 36; 358-361, 2002; Qin et al., Clin Chem. 2005; 51:75-83; Qin et al., Clin Chem 2006; 52:398-404.

D-dimer is notably described in Bayes-Genis et al., Am Heart J 2000; 140:379-84. Danesh et al., Circ. 2001; 103:2323-2327; Heim et al., Clinical Chemistry 2004; 50:1136-1147; Keeling et al. British Joumal of Haematology, 2004; 124:15-25; Shitrit et al., American Journal of Hematology 2004; 77:147-150; Shitrit et al. American Journal of Hematology 2004; 76:121-125.

CRP (C reactive protein) is notably described in Bermudez et al. Arterioscler Thromb Vasc Biol. 2002; 22:1668-1673; Body et al., International Journal of Cardiology 2005; 98:277-283; Ferreiro et al., Circ. 1999; 100:1958-1963; Haverkate et al., Lancet 1997; 349: 462-66; Lowe et al., Journal of Thrombosis and Hemostasis 2003; 1:2312-2316; Luc et al., Arterioscler Thromb Vasc Biol. 2003; 23:1255-1261; Maier et al. Circ. 2005; 111:1355-1361; Pepys et al., J. Clin. Invest. 2003 111:1805-1812; Ridker et al., Am Heart J 2004; 148:S19-26; Roberts et al. Clin Chem 2001; 47:418-425; Tanaka et al., J Am Coll Cardiol 2005; 45:1594-9; Tzoulaki et al. Circ. 2005; 112:976-983.

Fibrinogen is notably described in Saigo et al., Progress in Cardiovascular Diseases 2004; 46:524-538.

IMA (ischemia modified albumin) is notably described in Jaffe Cardiol Clin 2005 23:453-465.

FABP (fatty acid binding protein) is notably described in Suzuki et al. Int. Heart J 2005 46:601-606.

Myoglobin is notably described in Jaffe Cardiol Clin 2005 23:453-465. cTnl is notably described in Babuin et al., CMAJ 2005; 173:1191-1202; Bodi et al., International journal of Cardiology 2005; 98:277-283; Collinson et al., Ann clin biochem 2001; 38:423-449; Heeschen et al., Clin Biochem 2000; 33:359-368; Kamineni et al., Progress in cardiovascular diseases 2004; 46:379-392; Maier et al., Clin chim acta 1996; 45:19-38; McDonough et al., Progress in cardiovascular diseases 2004; 47:207-216; Mockel et al., Clin chim acta 2000; 293:139-155; Newby et al., Progress in cardiovascular diseases 2004; 46:404-416; Panteghini et al., Clin Chem Lab Med. 2001; 39:175-179. Rao et al. Am J cardiol 2003; 91:936-940; Ritter et al. Clin Chem. 2004; 50:112-119; Rosalki et al. Clin Chem 2004; 50:2205-2213; Scirica et al. Progress in cardiovascular diseases 2004; 47:177-188.

BNP (B natriuretic peptide), proBNP and NT-proBNP are notably described in Alehagen et al. International Journal of Cardiology 2005; 100:125-133; Bassan et al., European Heart Journal 2005; 26:234-240; De lemos et al., N Engl J Med 2001; 345:1014-21; de Lemos et al., Rev Cardiovasc Med 2003; 4:S37-S46; Fonarow et al., Rev Cardiovasc Med 2003; 4:S20-S28; Galvani et al., The European Journal of Heart Failure 2004; 6:327-333; Gibler et al., Rev Cardiovasc Med 2003; 4:S47-S55; Jernberg et al., European Heart Journal 2004; 25:1486-1493; Mega et al., J Am Coll Cardiol 2004; 44:335-9; Na Hong et al., Circ J 2005; 69:1472-1476; Sabatine et al., J Am Coll Cardiol 2004; 44:1988-95; Sokoll et al. Clin Chem Lab Med 2004; 42:965-972; Wiviott et al., Clin Chim Acta 2004; 346:119-128; Giuliani et al., Clinical Chemistry 2006; 52:1054-1061.

In a particularly preferred embodiment of the above defined prognosis anc diagnosis methods, the following combinations of biochemical markers are used:

| Combinations | Markers | | | | | | | |
|---|---|---|---|---|---|---|---|---|
| | MPO | sCD40L | IL-6 | PaPP-A | CRP | D-dimer | cTnl | proBNP |
| 1 | x | x | x | | | | | |
| 2 | x | x | | x | | | | |
| 3 | x | x | | | x | | | |
| 4 | x | x | | | | x | | |
| 5 | x | x | | | | | x | |
| 6 | x | x | | | | | | x |
| 7 | x | | x | x | | | | |
| 8 | x | | x | | x | | | |
| 9 | x | | x | | | x | | |
| 10 | x | | x | | | | x | |
| 11 | x | | x | | | | | x |
| 12 | x | | | x | x | | | |
| 13 | x | | | x | | x | | |
| 14 | x | | | x | | | x | |
| 15 | x | | | x | | | | x |
| 16 | x | | | | x | x | | |
| 17 | x | | | | x | | x | |
| 18 | x | | | | x | | | x |
| 19 | x | | | | | x | x | |
| 20 | x | | | | | x | | x |
| 21 | | x | x | x | | | | |
| 22 | | x | x | | x | | | |
| 23 | | x | x | | | x | | |
| 24 | | x | x | | | | x | |
| 25 | | x | x | | | | | x |
| 26 | | x | | x | x | | | |
| 27 | | x | | x | | x | | |
| 28 | | x | | x | | | x | |
| 29 | | x | | x | | | | x |
| 30 | | x | | | x | x | | |
| 31 | | x | | | x | | x | |
| 32 | | x | | | x | | | x |
| 33 | | x | | | | x | x | |
| 34 | | x | | | | x | | x |
| 35 | | | x | x | x | | | |
| 36 | | | x | x | | x | | |
| 37 | | | x | x | | | x | |
| 38 | | | x | x | | | | x |
| 39 | | | x | | x | x | | |
| 40 | | | x | | x | | x | |
| 41 | | | x | | x | | | x |
| 42 | | | x | | | x | x | |
| 43 | | | x | | | x | | x |
| 44 | | | | x | x | x | | |
| 45 | | | | x | x | | x | |
| 46 | | | | x | x | | | x |
| 47 | | | | x | | x | x | |
| 48 | | | | x | | x | | x |
| 49 | | | | | x | x | x | |
| 50 | | | | | x | x | | x |
| 51 | | | | | | x | x | x |

As intended herein, it is preferred for the above-defined prognostic and diagnostic methods that deduction of the probability that the patient will experience a vascular event or determining if the patient suffers from ACS, from the measured presence or level of the markers, is performed using a computer-based system. Even more preferably, the computer-based system is included in the automated assay system.

Depending on the deduced probability that the patient will experience a vascular event, it is within the frame of the present invention that several therapeutically decisions, such as hospitalization or pharmaceutical treatment, are offered to the practitioner using the method according to the invention. In particular if the deduced probability that the patient will experience a vascular event is considered negligible then it is proposed that the patient be sent home; on the contrary, if the deduced probability that the patient will experience a vascular event is considered high then it is proposed that the patient be admitted to hospital.

Probability that the patient will experience a vascular event can be assessed using scoring systems well known to the man skilled in the art.

Such scoring systems are notably described in Sabatine et al. (2002) Circ. 105:1760-1763. For instance, 0 or 1 is affected for each marker in the combination of markers used in the method according to the invention, depending on whether this marker is respectively absent or present or whether the level of this marker is respectively below or above a pre-determined cut-off value for normality, and a global sum is calculated for the combination of markers.

Alternatively, values between 0 and 1 can be used using several cut-off values such as described in WO 2004/059293. Besides, weighing factors can be affected to certain particular combinations of levels of markers which reflect a particular physiological setting.

The sum is then compared to predetermined values which are correlated to a probability that the patient will experience a vascular event. These predetermined values can be obtained according to statistical methods well known to the man skilled in the art, such as those described in US 2006/0105419 (see Example 3) or in EP 1 666 881 (see [0126] - [0143]). In particular, these predetermined values can obtained by using clinical studies wherein the occurrence of a vascular event in a patient within a determined follow-up period, or clinical end-point, is correlated to the level of one or more biochemical markers in said patient at the beginning of the follow-up period.

The biochemical markers which are used in the methods according to the invention are selected according to their statistical significance with respect to various differential analysis methods, such as T-Student test, ANOVA, or Kruskal-Wallis test. The markers can be further selected according to their discriminative power through a Receiver Operating Characteristic (ROC) curve analysis well-known to the man skilled in the art (Griner et al. Annals of Internal Medicine 1981; 94:555-600). Finally, the best markers are combined to increase their sensitivity (Se) and their specificity (Sp), for example, by following the general mROC method described by Kramar et al. Revue d'Epidémiologie et Santé Publique 1999; 47:376-383 or with unsupervised learning algorithms such as decision trees, Support Vector Machines (SVMs) or neural networks.

### EXAMPLE

### Clinical study

The objective of the clinical study is to make a statistic evaluation of the prognostic value of various combinations of markers by determining the sensibility, the specificity, the positive predictive value and the negative predictive value of each combination. The validation of the results is made by following the occurrence of vascular events in patients (pain recurrence, worsening of the patient's condition to unstable angina pectoris, non-fatal myocardial infarct, fatal myocardial infarct) 3 to 6 months following a first hospital admission for a chest pain.

The automated simultaneous determination in a single sample of the patient of these combinations of markers is particularly useful for the physician to help him establish risk stratifications for patients with chest pains and with no electrocardiogram (ECG) modification nor cardiac marker elevation.

### Patients

At least 300 patients consulting for chest pains and which show no elevation of the ST segment nor of cardiac Troponin I level on admission are enrolled in this study.

The following inclusion criteria are used:
- the patients are 18 years old and over and present with chest pains without elevation of the ST segment on the EGG performed on admission;
- the patients for whom cTnl assays performed on admission are negative.

Moreover, in order to validate the results a wide range of patients either with pathologies characteristic of Acute Coronary Syndrome or not are also selected:

50 myocardial infarcts: with typical prolonged pain and ECG modification and/or an elevation of the biochemical markers (e.g. cTnl).

100 healthy subjects, with no particular cardiovascular risk; this population is heterogeneous as regards age and sex.

Patients presenting risk factors are also included:
- Smokers,
- Patients with high arterial tension,
- Diabetics,
- Elderly patients,
- Patients with high triglyceride level.

However, patients admitted with the preceding characteristics but with pain for 12 hours or more are not included in the study. Patients with a non-normal ECG which cannot be used to visualize ischemic changes in the ST segment (e.g. pace maker) significant of a myocardial infarct or with an elevated level of cTnl on admission are not included in the study either.

Moreover, subjects with inflammatory diseases (CRP > 15 mg/l) and tumors, but also subjects having undergone surgery or a major trauma in the preceding month are not included in the study. Furthermore, patients having known thrombosis disorders or cardiovascular pathologies, or a myocardial infarct in the 3 weeks preceding admission are also excluded from the present study.

Patients with hepatic or renal disorders are not included in the study because of the risk of obtaining significantly elevated levels for the markers having a rapid renal clearance in healthy patients.

Pregnant women are also excluded from the study.

The following information on the included patients are collected: age, sex, smoking habits, diabetic status, pain characteristics (pain localization and strength, duration, recurrence), ECG characteristics on admission, admission time, cTnl assay results on admission, final diagnostic. Hormonal substitution treatment for menopaused women is also recorded.

### Sampling

For each patient, 2 ml of venous blood are collected in standard EDTA and dry tubes. Each tube is marked with a code identifying the patient, date and time of sampling, and time lapsed from admission to sampling.

### Assays

Assays for IL-6, HsCRP, MPO, PaPP-A, D-dimer, sCD40L and proBNP are conducted on the samples with methods known in the art.

The levels of the various markers for each patient will then be correlated to the putative occurrence of one or several vascular events in said patient, such as pain recurrence, worsening of the patient's condition to unstable angina pectoris, non-fatal myocardial infarct or fatal myocardial infarct, 3 to 6 months after sampling, according to statistical methods well known to the man skilled in the art, such as an analysis-of-variance (ANOVA). As such, it can be determined whether a significant relation exists between the studied variables (i.e. the studied clinical markers and the vascular events).

All the cited bibliographic references are incorporated herein by reference.

## Claims

1. A method for the prognosis of a vascular event in a patient suspected of being at risk for a vascular event, said patient presenting:
- no elevation of the ST segment as seen on an electrocardiogram, and/or
- a normal level of at least one myocardial necrosis marker,
wherein the presence and/or levels of at least two different biochemical markers are measured in a biological sample of said patient, said biochemical markers being selected from the group consisting of the following classes of markers:
- atherosclerotic plaque instability markers,
- atherosclerotic plaque disruption markers,
- coronary inflammation markers,
- pre-thrombotic status markers,
- thrombosis markers,
- ischemic markers,
- myocardial necrosis markers, and
- myocardial dysfunction markers,
whereby the probability that the patient will experience a vascular event is deduced from the measured presence and/or levels of the biochemical markers.

2. A method for the prognosis of a vascular event according to claim 1, wherein, the patient presents a chest pain.

3. A method for the prognosis of a vascular event according to claim 1 or 2, wherein the patient presents a normal cardiac Troponin level and/or a normal CK level.

4. A method for the prognosis of a vascular event according to any of claims 1 to 3, wherein:
- the atherosclerotic plaque instability marker is selected from the group consisting of MPO, sCD40L, IL-6, PaPP-A and choline;
- the atherosclerotic plaque disruption marker is selected from the group consisting of MPO, sCD40L, MMPs, Choline and PaPP-A;
- the coronary inflammation marker is selected from the group consisting of MPO, sCD40L, CRP and IL-6;
- the pre-thrombotic status marker is fibrinogen;
- the thrombosis marker is selected from the group consisting of D-dimers;
- the ischemic marker is selected from the group consisting of IMA, Cys-albumin, cTnl, cTnT, and FABP;
- the myocardial necrosis marker is selected from the group consisting of cTnl, cTnT, CK, Myo and FABP;
- the myocardial dysfunction marker is selected from the group consisting of BNP, NT-proBNP and proBNP.

5. A method for the prognosis of a vascular event according to any of claims 1 to 4, wherein the following combinations of biochemical markers are used:
| Combinations | Markers | | | | | | | |
|---|---|---|---|---|---|---|---|---|
| | MPO | sCD40L | IL-6 | PaPP-A | CRP | D-dimer | cTnl | proBNP |
| 1 | x | x | x | | | | | |
| 2 | x | x | | x | | | | |
| 3 | x | x | | | x | | | |
| 4 | x | x | | | | x | | |
| 5 | x | x | | | | | x | |
| 6 | x | x | | | | | | x |
| 7 | x | | x | x | | | | |
| 8 | x | | x | | x | | | |
| 9 | x | | x | | | x | | |
| 10 | x | | x | | | | x | |
| 11 | x | | x | | | | | x |
| 12 | x | | | x | x | | | |
| 13 | x | | | x | | x | | |
| 14 | x | | | x | | | x | |
| 15 | x | | | x | | | | x |
| 16 | x | | | | x | x | | |
| 17 | x | | | | x | | x | |
| 18 | x | | | | x | | | x |
| 19 | x | | | | | x | x | |
| 20 | x | | | | | x | | x |
| 21 | | x | x | x | | | | |
| 22 | | x | x | | x | | | |
| 23 | | x | x | | | x | | |
| 24 | | x | x | | | | x | |
| 25 | | x | x | | | | | x |
| 26 | | x | | x | x | | | |
| 27 | | x | | x | | x | | |
| 28 | | x | | x | | | x | |
| 29 | | x | | x | | | | x |
| 30 | | x | | | x | x | | |
| 31 | | x | | | x | | x | |
| 32 | | x | | | x | | | x |
| 33 | | x | | | | x | x | |
| 34 | | x | | | | x | | x |
| 35 | | | x | x | x | | | |
| 36 | | | x | x | | x | | |
| 37 | | | x | x | | | x | |
| 38 | | | x | x | | | | x |
| 39 | | | x | | x | x | | |
| 40 | | | x | | x | | x | |
| 41 | | | x | | x | | | x |
| 42 | | | x | | | x | x | |
| 43 | | | x | | | x | | x |
| 44 | | | | x | x | x | | |
| 45 | | | | x | x | | x | |
| 46 | | | | x | x | | | x |
| 47 | | | | x | | x | x | |
| 48 | | | | x | | x | | x |
| 49 | | | | | x | x | x | |
| 50 | | | | | x | x | | x |
| 51 | | | | | | x | x | x |

6. A method for the prognosis of a vascular event according to any of claims 1 to 5, wherein the vascular event is selected from the group consisting of myocardial vascular-related death, myocardial infarction, congestive-heart failure and vascular-related hospitalization, revascularization procedure.

7. A method for diagnosing acute coronary syndrome (ACS) in a patient presenting:
- no elevation of the ST segment as seen on an electrocardiogram, and/or
- a normal level of at least one myocardial necrosis marker,
comprising measuring the presence and/or levels of at least two different biochemical markers in a biological sample of said patient, said biochemical markers being selected from the group consisting of the following classes of markers:
- atherosclerotic plaque instability markers,
- atherosclerotic plaque disruption markers,
- coronary inflammation markers,
- pre-thrombotic status markers,
- thrombosis markers,
- ischemic markers,
- myocardial necrosis markers, and
- myocardial dysfunction markers,
whereby it is determined if said patient suffers from ACS.

8. A method for diagnosing ACS according to claim 7, wherein the patient presents a chest pain.

9. A method for diagnosing ACS according to claim 7 or 8, wherein the patient presents a normal cardiac Troponin level and/or a normal CK level.

10. A method for diagnosing ACS according to any of claims 7 to 9, wherein the biochemical markers are as defined in claim 4 or 5.

11. A kit intended for diagnosing ACS or for the prognosis of a vascular event in a patient, said kit comprising at least three ligands specific respectively to at least three biochemical markers selected from the group consisting of the following classes of markers:
- atherosclerotic plaque instability markers,
- atherosclerotic plaque disruption markers,
- coronary inflammation markers,
- pre-thrombotic status markers,
- thrombosis markers,
- ischemic markers,
- myocardial necrosis markers, and
- myocardial dysfunction markers.

12. A kit according to claim 11, wherein the biochemical markers are as defined in claim 4 or 5.

13. A kit according to claim 11 or 12, comprising one specific ligand to cardiac Troponin 1.

14. A kit according to any of claims 11 to 13, wherein the specific ligands are monoclonal antibodies.
